# EUROPEAN PATENT SPECIFICATION

(11) **EP 2 058 291 B1**
(45) Date of publication and mention of the grant of the patent: **28.11.2012**
(21) Application number: 07806415.1
(22) Date of filing: 30.08.2007
(51) Int. Cl.: C07C 51/41, C07C 59/135, C08F 2/26, C08F 14/26, C08F 114/26

(54) **PERFLUOROCARBOXYLIC ACID SALT AND METHOD FOR PRODUCING THE SAME**
PERFLUORCARBONSÄURESALZ UND VERFAHREN ZU DESSEN HERSTELLUNG
SEL D'ACIDE PERFLUOROCARBOXYLIQUE ET PROCÉDÉ DE FABRICATION

(30) Priority: 31.08.2006 JP 2006236515
(43) Date of publication of application: 13.05.2009
(73) Proprietor: Asahi Glass Company, Limited, Chiyoda-ku Tokyo 100-8405 (JP)
(72) Inventor: TAKAGI, Hirokazu, Chiyoda-ku Tokyo 100-8405 (JP); SEKI, Ryuji, Chiyoda-ku Tokyo 100-8405 (JP)
(74) Representative: Müller-Boré & Partner Patentanwälte
(86) International application number: PCT/JP2007/066938
(87) International publication number: WO 2008/026707

(56) References cited:
- EP-A1- 1 688 441
- WO-A1-2004/085492
- WO-A1-2006/127226
- GB-A- 1 311 896
- JP-A- 07 206 909
- JP-A- 2002 535 456
- JP-A- 2003 119 204
- US-A- 4 400 325
- US-A- 4 578 222

## Description

### TECHNICAL FIELD

The present invention relates to a a perfluorocarboxylic acid salt process for producing

### BACKGROUND ART

Heretofore, the surface tension reducing ability of a fluorine type surfactant has been known to be better than a hydrocarbon type surfactant having a corresponding structure or a silicone type surfactant. Therefore, the fluorine type surfactant is used in a broad field. Applications of the fluorine type surfactant include, for example, an emulsifier (a polymerization emulsifier) in emulsion polymerization, a leveling agent such as wax, a blowing aid, an additive for forming stable foams for foam fire extinguishing and improving fire-extinguishing performance, a cleaner, a mold releasing agent, an anticorrosive agent, a latex stabilizer, an anti-fogging agent for films for agriculture, a pigment dispersant, an agent to improve wettability or permeability of e.g. ink, coating material, resist, an agent to impart water and oil repellency to a curable resin, an antifouling agent.

The fluorine type surfactant is a compound having a structure wherein a hydrophilic group is bonded to a hydrophobic group having a fluoroalkyl skeleton. The surface tension reducing ability of the fluorine type surfactant depends on a fluorination percentage of the fluoroalkyl skeleton, and the higher the fluorination percentage, the higher the surface tension reducing ability. Therefore, it is considered that the fluorine type surfactant is preferably one having a perfluorinated fluoroalkyl skeleton.

In the past, fluorinated compounds having various structures useful as surfactants, have been proposed, and as one of such fluorinated compounds, a perfluorocarboxylic acid salt containing an etheric oxygen atom may be mentioned.

As a process for obtaining the perfluorocarboxylic acid salt, a process has been known, wherein a perfluorocarboxylic acid fluoride obtained by ring-opening polymerization of tetrafluoroethylene oxide or hexafluoropropylene oxide, is hydrolyzed and converted to a perfluorocarboxylic acid, and such an acid is further converted to a salt such as an ammonium salt or an alkali metal salt, and it is reported that the obtained perfluorocarboxylic acid salt is used as a surfactant (cf. Patent Document 1).

Patent Document 1: U.S. Patent No. 3,271,341

### DISCLOSURE OF THE INVENTION

### OBJECTS TO BE ACCOMPLISHED BY THE INVENTION

However, the perfluorocarboxylic acid salt obtained by the above-mentioned conventional process, has a problem such that the surface tension reducing ability is not sufficient, or that every product has a different surface tension reducing ability or may be colored. Such a problem is likely to lead to a deterioration of the quality of a product to be produced by using the perfluorocarboxylic acid salt.

The present invention has been made under the above circumstances, and it provides a process for producing perfluorocarboxylic acid salt excellent in the surface tension reducing ability it.

### MEANS TO ACCOMPLISH THE OBJECTS

By a study made by the present inventors, it has been found that the perfluorocarboxylic acid salt obtained by the above-mentioned conventional process, contains a large amount of metals as impurities. Further, they have found that a certain specific metal presents an adverse effect on the surface tension reducing ability of the perfluorocarboxylic acid salt, and also that if the production of a fluorinated polymer is carried out by using the perfluorocarboxylic acid salt containing such a metal in an amount of at least a certain level, the resulting polymer tends to be colored.

The present invention provides the following.

A process for producing the perfluorocarboxylic acid salt having an iron content of at most 10 ppm by mass and represented by the following formula (1):

R^{F}(OCF(X¹)CF₂)ₖ₁lOCF(X²)COO⁻M⁺ (1)

wherein R^{F} is a C₁₋₁₀ monovalent perfluorinated organic group, each of X¹ and X² is independently a fluorine atom or a trifluoromethyl group, k is an integer of at least 1, and M⁺ is an ammonium ion or an alkyl-substituted ammonium ion which comprises a hydrolysis step of hydrolyzing a compound (2) represented by the following formula (2) with water by using a reaction apparatus equipped with a reactor in which at least its inner surface is made of a fluororesin, wherein the amount of water to be used is from 0.9 to 1.2 times by mol based on the compound (2), wherein the hydrofluoric acid content in the reaction product is adjusted to be at most 0.1 mass% and then the obtained reaction product is subjected to distillation for purification, to obtain a compound (3) represented by the following formula (3), and a salt-forming step of the compound (3) to obtain the perfluorocarboxylic acid salt:

R^{F} (OCF (X¹) CF₂)ₖ₋₁OCF(X²) COF (2)

R^{F}(OCF(X¹)CF₂)ₖ₋₁OCF(X²)COOH (3)

wherein R^{F}, X¹, X² and k are respectively the same as R^{F}, X¹, X² and k in the formula (1).

The process for producing the perfluorocarboxylic acid salt, wherein the hydrolysis is carried out by reacting the compound (2) without a solvent.

The process for producing the perfluorocarboxylic acid salt, wherein the formed hydrofluoric acid is discharged out of the reaction system by bubbling nitrogen in a hydrolysis reaction crude liquid.

The process for producing the perfluorocarboxylic acid salt, wherein X¹ and X² in the formulae (1), (2) and (3) are both fluorine atoms.

The process for producing the perfluorocarboxylic acid salt wherein the total content of sodium and potassium is at most 50 ppm by mass.

The process for producing the perfluorocarboxylic acid salt a wherein the total content of calcium and magnesium is at most 10 ppm by mass.

The process for producing the perfluorocarboxylic acid salt, wherein the total content of iron, sodium, potassium, calcium, magnesium, chromium and nickel is at most 70 ppm by mass.

### EFFECTS OF THE INVENTION

According to the present invention, it is possible to provide a perfluorocarboxylic acid salt excellent in surface tension reducing ability. As a result, when such a perfluorocarboxylic acid salt is used as a surfactant for polymerization of tetrafluoroethylene, it is possible to obtain a polymer at a high yield. Further, a tetrafluoroethylene polymer which is produced by using the perfluorocarboxylic acid salt has no problem of coloration.

### PROCESS FOR PRODUCTION OF PERFLUOROCARBOXYLIC ACID SALT

The perfluorocarboxylic acid salt of the present invention can be produced via, for example, a step of hydrolyzing the following compound (2) to obtain the following compound (3), and a step of converting the compound (3) to a salt to obtain the compound (1):

R^{F}(OCF(X¹) CF₂)_{K-1}OCF(X²)COF (2)

R^{F}(OCF(X¹)CF₂)ₖ₋₁OCF(X²) COOH (3)

wherein R^{F}, X¹, X² and k are respectively the same as R^{F}, X¹, X² and k in the formula (1).

Specific examples of the compound (2) may be the following compounds.

CF₃OCF₂CF₂OCF₂COF,

CF₃CF₂CF₂OCF₂CF₂OCF₂COF,

CF₃ (CF₂) ₃OCF₂CF₂OCF₂COF ,

CF₃ (OCF₂CF₂)₂OCF₂COF,

CF₃CF₂OCF₂CF₂OCF₂COF,

CF₃CF₂ (OCF₂CF₂) ₂OCF₂COF, and

CF₃CF₂CF₂ (OCF₂CF₂) ₂OCF₂COF.

Specific examples of the compound (3) may be the following compounds.

CF₃OCF₂CF₂OCF₂COOH,

CF₃ (OCF₂CF₂) ₂OCF₂COOH,

CF₃CF₂OCF₂CF₂OCF₂COOH,

CF₃ (CF₂) ₃OCF₂CF₂OCF₂COOH,

CF₃CF₂ (OCF₂CF₂) ₂OCF₂COOH, and

CF₃CF₂CF₂ (OCF₂CF₂) ₂OCF₂COOH.

As the compound (2), it is possible to use a commercially-available one, or the compound (2) may be produced by using a known compound in accordance with a known process. The process may, for example, be a process described in WO00/56694 by the present applicant, wherein the following compound (p1) is used as a raw material. In the process, the following compound (p1) and the following compound (p2) are subjected to an esterification reaction to obtain the following compound (p3), the compound (p3) is perfluorinated to obtain the following compound (p4), and an ester bond in the compound (p4) is subjected to a decomposition reaction to obtain the compound (2) (perfluorocarboxylic and fluoride).

R(OCH(X³)CH₂)ₖOH (p1)

Q(COF)ₙ (p2)

Q[COO(CH₂CH(X³)O)ₖR]ₙ (p3)

Q^{f}[COO(CF₂CF(X⁴)O)ₖR^{F}]ₙ (p4)

R^{F}(OCF(X¹)CF₂)ₖ₋₁OCF(X²)COF (2)

wherein R^{F}, X¹, X² and k are respectively the same as R^{F}, X¹, X² and k in the formula (1). R is a C₁₋₁₀ monovalent organic group having a portion to be fluorinated, X³ is a hydrogen atom or a methyl group, Q is an n valent fluorinated organic group, n is an integer of from 1 to 4, Q^{f} is the same as Q when Q is a fluorinated organic group having no portion to be fluorinated and is a perfluorinated Q when Q is a fluorinated organic group having a portion to be fluorinated, and X⁴ is a fluorine atom or a trifluoromethyl group.

The C₁₋₁₀ monovalent organic group for R may be, one mentioned as an organic group having a portion to be fluorinated, for the R^{F}, for example, a saturated hydrocarbon group, an etheric oxygen atom-containing saturated hydrocarbon group, a partially-halogenated saturated hydrocarbon group or a partially-halogenated (etheric oxygen atom-containing saturated hydrocarbon) group.

Q may be one having a structure wherein an n valent organic group having a portion to be fluorinated is fluorinated.

The n valent organic group having a portion to be fluorinated may, for example, be the same one as mentioned as the organic group having a portion to be fluorinated for the R^{F}, except that it has an n valence. For example, an n valent saturated hydrocarbon group, an n valent etheric oxygen atom-containing saturated hydrocarbon group, an n valent partially-halogenated saturated hydrocarbon group or an n valent partially-halogenated (etheric oxygen atom-containing saturated hydrocarbon) group, may be mentioned.

The n valent saturated hydrocarbon group may be a group wherein n hydrogen atoms in a saturated hydrocarbon (e.g. alkane or cycloalkane) became linkages. For example, a divalent saturated hydrocarbon group may be an alkylene group.

The n valent etheric oxygen atom-containing hydrocarbon group may be a group wherein n hydrogen atoms in the saturated hydrocarbon containing an etheric oxygen atom became linkages. The number of etheric oxygen atoms in the group is not particularly limited, and it may be one or at least two. For example, the divalent group may be an alkylene oxyalkylene group or an oxyalkylene group. In Q, in the organic group having portions to be fluorinated, the portions to be fluorinated can be partially fluorinated or entirely fluorinated (namely, it may be perfluorinated).

n is preferably an integer of from 1 to 4, more preferably an integer of from 1 to 2, most preferably 1. That is, Q is most preferably a monovalent fluorinated organic group.

The monovalent fluorinated organic group may, for example, be a perfluoroalkyl group such as CF₃(CF₂)ₐ- (a is preferably an integer of from 1 to 8) or CF₃CF(CF₃)CF₂CF₂-; or an etheric oxygen atom-containing perfluoroalkyl group such as CF₃CF₂CF₂OCF(CF₃) -, CF₃CF₂CF₂OCF (CF₃) CF₂OCF(CF₃) - or CF₃CF₂OCF₂CF₂OCF₂- .

Q^{f} is the same as Q when Q is a fluorinated organic group having no portion to be fluorinated, or perfluorinated Q when Q is a fluorinated organic group having a portion to be fluorinated.

That is, when the Q is one wherein in the organic group having a portion to be fluorinated, the portion to be fluorinated is perfluorinated, the compound (p3) does not change even if it is perfluorinated since Q has no more portion to be fluorinated.

On the other hand, when the Q is one wherein in the organic group having a portion to be fluorinated, the portion to be fluorinated is partially fluorinated, Q has more portion to be fluorinated, whereby in the step of perfluorinating the compound (p3), the portion to be fluorinated in Q is perfluorinated.

The hydrolysis reaction of the compound (2) can be carried out by a known method, and the condition, operation, means, of the reaction are not particularly limited.

For example, when the hydrolysis is carried out by reacting the compound (2) with water, the reaction temperature is preferably from -10 to +100°C, particularly preferably from 0 to 50°C. The reaction pressure is preferably at normal pressure, and it is possible to use a reduced pressure, atmospheric pressure, an elevated pressure.

The reaction is preferably carried out without a solvent, and depending on the melting point of a product, the reaction may be carried out in an inert solvent.

The amount of water to be used is preferably from 0.9 to 1.2 times by mol, particularly preferably from 0.95 to 1.05 times by mol, based on the compound (2).

The reaction time can suitably be changed by the reaction temperature, and it is preferably from 1 to 5 hours.

In the hydrolysis reaction, hydrofluoric acid (HF) is formed. The HF can be discharged out of the reaction system by bubbling nitrogen in a hydrolysis reaction crude liquid to let HF be entrained in the nitrogen stream.

The obtained reaction product can be used in the next salt-forming step as it is, but if necessary, it may be subjected to distillation for purification. By carrying out distillation for purification, it is possible to remove HF remaining in the reaction product. If HF remains in the reaction product, in a case where in a reaction apparatus used in a salt-forming step and in a distillation apparatus used in distillation for purification, if the material at a portion which contacts with the reaction product, contains a metal, the material is corroded by HF, and the metal content is eluted, whereby as a result, the metal content in the resulting product increases.

In order to suppress elution of the metal content, the amount of HF to be contained in the reaction product is adjusted to be preferably at most 1 mass%, more preferably at most 0.1 mass%, especially preferably at most 0.01 mass%, in the total mass of the reaction product, at a stage before the salt-forming step is carried out.

The salt-forming reaction of the compound (3) can be carried out by a known method to be used for neutralizing a carboxylic acid, and it can be carried out, for example, by neutralizing the compound (3) by adding an alkali aqueous solution thereto.

As the alkali, it is possible to use one corresponding M⁺ of the compound (1), and it may, for example, be ammonia, tetramethylammonium hydroxide, monoethylamine, diethylamine or triethylamine.

The aqueous medium to be used for producing the alkali aqueous solution may be the same one as mentioned as the aqueous medium which may be contained in the perfluorocarboxylic acid salt of the present invention.

The salt-forming reaction may be carried out in accordance with a common method, and its condition, operation, means, are not particularly limited. The reaction temperature is preferably from -10 to +100°C, particularly preferably from 0 to +50°C. The reaction pressure is preferably normal pressure, and it is possible to use a reduced pressure, atmospheric pressure, an elevated pressure.

The reaction product obtained in such a manner is an aqueous composition containing the compound (1). Such an aqueous composition may be used as it is, as the perfluorocarboxylic acid salt composition, and if necessary, it may be dried and used as a solid. When it is used as it is, the pH of the aqueous composition is preferably adjusted to be from 2 to 12, particularly preferably from 4 to 10, as mentioned above.

When M⁺ is an ammonium ion, the salt-forming reaction of the compound (3) may be carried out by blowing an ammonia gas into a solution wherein the compound (3) is dissolved in a nonaqueous solvent inert to the reaction. In such a case, the reaction product precipitates as a solid in the reaction solution, whereby it may be isolated by filtration.

The nonaqueous solvent to be used for the reaction may be one inert to the reaction, and it is preferred to use a chlorinated solvent such as dichloromethane and/or a fluorinated solvent such as dichloropentafluoropropane.

The reaction temperature is preferably from -10 to +100°C, particularly preferably from 0 to +50°C. The reaction pressure is at normal pressure, and it is possible to use a reduced pressure, atmospheric pressure, an elevated pressure.

The isolated reaction product contains the compound (1). The reaction product may be used as it is in the form of a solid, or it may be used in the form of an aqueous composition as dissolved in an aqueous medium. For the above reason, the reaction product is preferably used in the form of an aqueous composition, and in such a case, the pH is preferably adjusted to be from 2 to 12, particularly preferably from 4 to 10, in the same manner as above.

In the present invention, the iron content in the perfluorocarboxylic acid salt obtained in such a manner, is adjusted to be at most 10 ppm by mass. As such a method, it is preferred to use a method wherein in a step of hydrolyzing the compound (2), the hydrolysis is carried out by using a reaction apparatus equipped with a reactor in which at least its inner surface is made of a fluororesin. That is, the perfluorocarboxylic acid salt composition of the present invention is preferably one obtained via a step of hydrolyzing the compound (2) by using a reaction apparatus equipped with a reactor in which at least its inner surface is made of a fluororesin, to obtain the compound (3), and a salt-forming step of the compound (3) to obtain the compound (1).

By using the above reaction apparatus, in the step of hydrolysis, it is possible to easily and effectively suppress an increase of each metal content in the reaction product.

That is, in the step of hydrolyzing the compound (2), HF in a molar amount equal to the compound (3) is formed. HF has high corrosivity, and when a metal reactor or a glass reactor is used, a metal content such as iron, chromium, nickel, silicon, aluminum, copper, sodium, barium, boron, potassium, calcium, zinc, manganese, molybdenum or magnesium, is eluted from the reactor material. Further, when HF and water are coexistent in the reaction system, the corrosivity becomes remarkably high, and the elution amount of the metal content increases.

In the above-mentioned Patent Document 1, when a perfluorocarboxylic acid fluoride (corresponding to the compound (2)) is to be hydrolyzed, excess water is added to the perfluorocarboxylic acid fluoride, which is a hydrolyzing condition where corrosivity is remarkably high. On the other hand, in the Patent Document, there is no description relating to the type of a reactor material or a purification step for removing a metal content, and it is considered that a large amount of metal is contained in the perfluorocarboxylic acid salt to be obtained. According to the present inventors' findings, if a large amount of metal is coexistent with the compound (1), the surface tension reducing ability of the compound (1) is deteriorated, and the stability also decreases.

The above-mentioned reactor in which at least its inner surface is made of a fluororesin may, for example, be a reactor entirely made of a fluororesin or a reactor wherein the inner surface of the reactor made of a material other than a fluororesin, is coated with a fluororesin (hereinafter referred to as a fluororesin lining reactor). The reactor has a high corrosion resistance against HF, and it can prevent corrosion of the reactor by HF formed during the hydrolysis and elution of the metal content accompanying it, even under such a condition that water and HF are coexistent and the corrosivity is very high, and it can prevent an increase of the content of metal which does not constitute the compound (1).

The fluororesin may, for example, be polytetrafluoroethylene (PTFE), a tetrafluoroethylene/ethylene copolymer (COP), a tetrafluoroethylene/perfluoro(alkyl vinyl ether) copolymer (PFA), a tetrafluoroethylene/hexafluoropropylene copolymer (FEP), an ethylene/tetrafluoroethylene copolymer (ETFE), polychlorotrifluoroethylene (PCTFE), an ethylene/chlorotrifluoroethylene copolymer (ECTFE), polyvinylidene fluoride (PVDF) or polyvinyl fluoride (PVF). The fluororesin is preferably PTFE, COP or PFA.

The reaction apparatus may be equipped with an attendant equipment other than the reactor. A gas-contact portion or a liquid-contact portion of the attendant equipment may be a stirring blade, a condenser or a piping. In the present invention, to minimize elution of a metal content, it is preferred to use a reaction apparatus wherein not only a reactor but also the entire attendant equipment is coated with a fluororesin.

### METHOD OF PRODUCING HOMOPOLYMER OR COPOLYMER OF TETRAFLUOROETHYLENE

In the present invention, by using the perfluorocarboxylic acid salt as a polymerization emulsifier, emulsion polymerization of tetrafluoroethylene alone or together with another copolymerizable monomer is carried out in an aqueous medium.

Here, the polymerization emulsifier is preferably added from 0.1 to 10 parts by mass per 100 parts by mass of the total amount of the monomers.

When tetrafluoroethylene is polymerized alone, polytetrafluoroethylene (PTFE) is obtained.

Another monomer copolymerizable with tetrafluoroethylene may, for example, be a fluoromonomer such as vinylidene fluoride, hexafluoropropylene, chlorotrifluoroethylene or a perfluoro(alkyl vinyl ether) represented by CF₂=CFOR_{f} (wherein R_{f} is a C₁₋₁₆ perfluoroalkyl group which may contain an etheric oxygen atom).

By copolymerizing tetrafluoroethylene with such another monomer, it is possible to obtain a fluororesin such as polyvinylidene fluoride (PVDF), a tetrafluoroethylene/hexafluoropropylene copolymer (FEP) or a tetrafluoroethylene/perfluoro(alkyl vinyl ether) copolymer (PFA) .

Due to its excellent chemical resistance and high purity, such a fluororesin is widely used in the production process for semiconductors which requires high cleanliness. In recent years, high integration and refinement of semiconductor devices are in an acceleration state, and to realize more refined devices, it is required to reduce metal impurities included in the production process for semiconductor devices as much as possible. From such a viewpoint, the fluororesin obtained by the production method of the present invention can be said as preferred.

### EXAMPLES

Now, the present invention will be described in further detail with reference to Examples, but it should be understood that the present invention is by no means restricted thereto.

Further, hereinafter, 1,1,2-trichloro-1,2,2-trifluoroethane is referred to as R-113.

A gas chromatography is referred to as GC, and the results by a GC analysis is shown by a peak area ratio.

A gas chromatography mass spectrometry is referred to as GC-MS.

A nuclear magnetic resonance is referred to as NMR.

In the following Production Examples, room temperature means from 20 to 30°C.

The purity of a perfluorocarboxylic acid salt in the product (sample) produced in each of Examples of the present invention and Comparative Examples, was measured in such a manner that after the sample was subjected to methyl-esterification, a GC analysis was carried out.

Further, the content of each metal in a product produced in each of Examples of the present invention and Comparative Examples, was measured by an ICP method with respect to a sample solution prepared in such a manner that after a sample was incinerated, it was dissolved in a nitric acid aqueous solution.

### (A-1) PRODUCTION EXAMPLE OF CF₃CF₂OCF₂CF₂OCF₂COO⁻(NH₄)⁺ EXAMPLE 1

### STEP 1-1: PRODUCTION STEP OF

CH₃CH₂O(CH₂)₂O(CH₂)₂OCOCF(CF₃)OCF₂CF(CF₃)OCF₂CF₂CF₃ BY

### ESTERIFICATION REACTION

Into a 2 L Hastelloy C autoclave, 300 g of CH₃CH₂O(CH₂)₂O(CH₂)₂OH was introduced, and the autoclave was cooled. Under sealed-stirring, 1,339 g of CF₃CF₂CF₂OCF(CF₃)CF₂OCF(CF₃)COF was gradually introduced so that the inner temperature was kept to be at most 30°C. After the total amount of it was introduced, stirring was further carried out at 30°C for 3 hours, and then, HF formed by the reaction was removed out of the system by bubbling of a nitrogen gas to obtain a product.

As a result of the GC analysis on the product, CH₃CH₂O(CH₂)₂O(CH₂)₂OCOCF(CF₃)OCF₂CF(CF₃)OCF₂CF₂CF₃ was formed with a yield of 99.6%, and no unreacted CH₃CH₂O(CH₂)₂O(CH₂)₂OH was detected. Such a product was used in the next STEP 1-2 without purification.

### STEP 1-2: PRODUCTION STEP OF

CF₃CF₂O(CF₂)₂O(CF₂)₂OCOCF(CF₃)OCF₂CF(CF₃)OCF₂CF₂CF₃ BY

### FLUORINATION REACTION

Into a 500 mL nickel autoclave, 312 g of R-113 was added, followed by stirring to maintain the autoclave at 25°C. At the autoclave gas outlet, a cooler kept at 20°C, a NaF pellet-packed bed and a cooler kept at -10°C were set in series. Further, to the cooler kept at -10°C, a liquid-returning line was set to return a condensed liquid to the autoclave.

Into the autoclave, nitrogen gas was blown at room temperature for 1 hour, and then, a fluorine gas diluted to 20 vol% with nitrogen gas (hereinafter referred to as a 20% diluted fluorine gas) was blown therein at room temperature at a flow rate of 17.04 L/hr for 1 hour. Then, while blowing the 20% diluted fluorine gas therein at the same flow rate, a solution having 10 g of the product obtained in STEP 1-1 dissolved in 150 g of R-113, was injected therein over 4.1 hours.

Then, while blowing the 20% diluted fluorine gas therein at the same flow rate, the autoclave inner pressure was raised to 0.15 MPaG. Then, into the autoclave, a R-113 solution having a benzene concentration of 0.01 g/mL, was injected in an amount of 9 mL while increasing the temperature from 25°C to 40°C, and a benzene solution inlet of the autoclave was closed, followed by stirring continuously for 0.3 hour.

Then, while maintaining the autoclave inner pressure at 0.15 MPaG and the autoclave inner temperature at 40°C, the R-113 solution having a benzene concentration of 0.01 g/mL was injected in an amount of 6 mL, and the benzene solution inlet of the autoclave was closed, followed by stirring continuously for 0.3 hour. Further, the same operation was repeated once. The total injected amount of benzene was 0.22 g, and the total injected amount of R-113 was 21 mL.

Further, while blowing the 20% diluted fluorine gas therein at the same flow rate, stirring was continued for 1 hour. Then, the autoclave inner pressure was adjusted to normal pressure, and nitrogen gas was blown therein for 1 hour.

As a result of ¹⁹F-NMR analysis on the product, the above-identified compound was contained with a yield of 99%. Further, as analytical results of ¹H-NMR and GC-MS, no compound having a C-H bond was detected. STEP 1-3: PRODUCTION STEP OF CF₃CF₂O(CF₂)₂OCF₂COF BY DECOMPOSITION REACTION OF ESTER BOND

To the bottom (volume: 2 L) of a distillation column equipped with a refluxing device at 10°C, 4,273 g of CF₃CF₂O(CF₂)₂O(CF₂)₂OCOCF(CF₃)OCF₂CF(CF₃) OCF₂CF₂CF₃ was introduced, and 12.6 g of potassium fluoride was added thereto. While carrying out heating and stirring (heat medium temperature: 100 to 130°C) for 12 hours, a decomposition reaction was carried out. Then, distillation was carried out, and 1,273 g of a fraction having a purity of at least 99% was recovered as a main fraction.

Such a fraction had a boiling point of 66.5°C and a yield of 84.5%. Further, as analytical results of ¹H-NMR and GC-MS on the fraction, no compound having a C-H bond was detected. The analytical results of ¹⁹F-NMR on the product are as follows.

¹⁹F-NMR (282.7 MHz, solvent: CDCl₃, standard: CFCl₃) δ (ppm): 16.3(1F), -75.4(2F), -85.5(3F), -86.5(2F), -87.4(4F)

### STEP 1-4: PRODUCTION STEP OF CF₃CF₂O(CF₂)₂OCF₂COOH BY HYDROLYSIS

Into a 200 mL fluororesin lining reactor, 133 g of CF₃CF₂O(CF₂)₂OCF₂COF obtained in STEP 1-3 was introduced, and with cooling with ice, 6.7 g of water was slowly dropwisely added thereto while intensively stirring to carry out hydrolysis. After the dropwise addition, the temperature gradually rose to room temperature, and stirring was further continued for 5 hours. Then, while HF formed by the reaction was removed out of the system by bubbling nitrogen gas, stirring was carried out for further 15 hours.

As a result, CF₃CF₂O(CF₂)₂OCF₂COOH having a purity of 99.2% was obtained with a yield of 94%. Further, the amount of HF contained in the obtained CF₃CF₂O(CF₂)₂OCF₂COOH, was analyzed by an F ion meter, and it was 77 ppm by mass. The analytical results of the ¹⁹F-NMR on the product are as follows.

¹⁹F-NMR (282.7 MHz, solvent: CDCl₃, standard: CFCl₃) δ (ppm) : -78.7(2F), -87.3(3F), -88.8(2F), -89.1(4F). STEP 1-5: PRODUCTION STEP OF CF₃CF₂OCF₂CF₂OCF₂COO⁻(NH₄)⁺ BY AMMONIUM SALT-FORMING

Into a 200 mL glass reactor equipped with a stirrer and a reflux condenser, 58 g of 2.8 mass% aqueous ammonia prepared by diluting commercially-available aqueous ammonia (28 mass%) by ten times, was introduced. Then, while stirring it, 33 g of CF₃CF₂O(CF₂)₂OCF₂COOH obtained in STEP 1-4 was slowly dropwisely added thereto with cooling with ice. After completion of the dropwise addition, the temperature was gradually increased and was kept at 40°C for 5 hours. After that, while measuring the solid concentration (measured by evaporation to dryness) and the pH (measured by a pH meter), 2.8 mass% aqueous ammonia and water were added little by little, followed by stirring to adjust the solid concentration=30.0% and pH=5.7, whereby 115 g of a CF₃CF₂O(CF₂)₂OCF₂COO⁻ (NH₄)⁺ aqueous solution was obtained.

A part of the aqueous solution was evaporated to dryness, and the purity of the obtained solid was measured in accordance with the above-mentioned measuring method and was 99%. Further, the metal content contained in the solid was analyzed by an ICP method. The analytical results are shown in Table 1.

### COMPARATIVE EXAMPLE 1

115 g of a CF₃CF₂O(CF₂)₂OCF₂COO⁻ (NH₄)⁺ aqueous solution (solid concentration: 30.0% and pH: 5.7) was obtained in the same manner as in Example 1 except that the fluororesin lining reactor used in STEP 1-4 of Example 1 was changed to a SUS304 reactor, and that 6.7 g of water was changed to 10.0 g of water.

A part of the aqueous solution was evaporated to dryness, and the metal content was analyzed in the same manner as in Example 1. The analytical results are shown in Table 1.

### EXAMPLE 2

115 g of a CF₃CF₂O(CF₂)₂OCF₂COO⁻(NH₄)⁺ aqueous solution (solid concentration: 30.0% and pH: 5.7) was obtained in the same manner as in Example 1 except that the glass reactor used in STEP 1-5 of Example 1 was changed to a SUS304 reactor.

A part of the aqueous solution was evaporated to dryness, and the metal content was analyzed in the same manner as in Example 1. The analytical results are shown in Table 1.

### (A-2) PRODUCTION EXAMPLE OF CF₃(CF₂)₃O(CF₂)₂OCF₂COO⁻(NH₄)⁺ EXAMPLE 3

A CF₃(CF₂)₃O(CF₂)₂OCF₂COO⁻(NH₄)⁺ aqueous solution (solid concentration: 30.0% and pH: 5.7) was obtained in the same manner as in Example 1 except that CH₃CH₂O(CH₂)₂O(CH₂)₂OH used in STEP 1-1 of Example 1 was changed to CH₃(CH₂)₃O(CH₂)₂O(CH₂)₂OH.

A part of the aqueous solution was evaporated to dryness, and the metal content was analyzed in the same manner as in Example 1. The analytical results are shown in Table 1.

**TABLE 1**

| | | Ex. 1 | Ex. 2 | Ex. 3 | Comp. Ex. 1 |
|---|---|---|---|---|---|
| Metal content (ppm by mass) | Fe | 0.6 | 1.8 | 4.1 | 46.6 |
| | Cr | 0.1 | 1.8 | 1.7 | 21.4 |
| | Na | 2.4 | 1.7 | 19.8 | 67.3 |
| | Al | 0.1 | 0.7 | 0.8 | 28.8 |
| | Ba | 0.5 | 0.3 | 5.3 | 14.8 |
| | Cu | 0.0 | 0.2 | 0.3 | 24.4 |
| | B | 0.4 | 0.2 | 7.4 | 16.3 |
| | Ni | 0.1 | 0.2 | 0.7 | 5.9 |
| | K | 0.4 | 0.2 | 4.7 | 9.6 |
| | Ca | 0.4 | 0.2 | 4.4 | 10.4 |
| | Zn | 0.0 | 0.0 | 0.4 | 2.2 |
| | Mn | 0.0 | 0.0 | 0.1 | 1.5 |
| | Mg | 0.0 | 0.0 | 0.2 | 0.7 |
| Sum of contents of iron, sodium, potassium, calcium, magnesium, chromium and nickel | Sum | 4.1 | 6.0 | 35.7 | 162.0 |
| Sum of contents of iron, chromium, nickel, silicon, aluminum, copper, sodium, barium, boron, potassium, calcium, zinc, manganese and magnesium in sample as measured by ICP method | Sum | 5.2 | 7.6 | 50.0 | 250.0 |

### (B) PREPARATION OF PERFLUOROCARBOXYLIC ACID SALT AQUEOUS SOLUTION AND MEASUREMENT OF SURFACE TENSION

### TEST EXAMPLE 1

Into the CF₃CF₂O (CF₂)_{2O}CF₂COO⁻ (NH₄)⁺ aqueous solution obtained in each of Examples 1 to 3 and Comparative Example 1, 2.8 mass% aqueous ammonia and water were added to have the solid concentration and pH as shown in Table 2, followed by stirring, whereby Example samples 1 to 15 and Comparative Example samples 1 to 3 as shown in Table

### 1 were prepared.

Then, with respect to each sample, its properties (appearance and physical properties) at a solid concentration of 30 mass% and its surface tension reducing ability at a solid concentration of 0.1 mass% and 1.0 mass%, were evaluated. The surface tension reducing ability was evaluated by measuring the surface tension by using a platinum plate and by a wilhelmy method. The smaller the surface tension, the larger the surface tension reducing ability. The evaluation results are shown in Table 2. Further, in Table 2, the metal content of carboxylic acid salt is a sum of the contents of iron, chromium, sodium, aluminum, barium, copper, boron, nickel, potassium, calcium, zinc, manganese and magnesium.

**TABLE 2**

| | | Perfluorocarboxylic acid salt aqueous solution used | | pH | Solid concentration (mass%) | Properties | Surface tension (mN/m) |
|---|---|---|---|---|---|---|---|
| | | | Metal content of carboxylic acid salt (ppm by mass) | | | | |
| Example samples | 1 | Ex. 1 | 5 | 1.8 | 0.1 | Clear/liquid | 51 |
| | 2 | | | | 1.0 | Clear/gel | 29 |
| | 3 | | | | 30.0 | | - |
| | 4 | | | 6.8 | 0.1 | Clear/liquid | 59 |
| | 5 | | | | 1.0 | | 39 |
| | 6 | | | | 30.0 | | - |
| | 7 | | | 9.6 | 0.1 | Clear/liquid | 59 |
| | 8 | | | | 1.0 | | 39 |
| | 9 | | | | 30.0 | | - |
| | 10 | | | 10.6 | 0.1 | Clear/liquid | 59 |
| | 11 | | | | 1.0 | | 39 |
| | 12 | | | | 30.0 | | - |
| | 13 | Ex. 2 | 7.6 | 6.8 | 0.1 | Clear/liquid | 59 |
| | 14 | | | | 1.0 | | 39 |
| | 15 | | | | 30.0 | | - |
| | 16 | Ex. 3 | 50 | 6.8 | 0.1 | Clear/liquid | 59 |
| | 17 | | | | 1.0 | | 39 |
| | 18 | | | | 30.0 | | - |
| Comparative Example samples | 1 | Comp. Ex. 1 | 250 | 6.8 | 0.1 | Clear/liquid | 65 |
| | 2 | | | | 1.0 | Light brown/liquid | 45 |
| | 3 | | | | 30.0 | | - |

As apparent from the above results, each of the Example samples 1 to 15 which used a CF₃CF₂O(CF₂)₂OCF₂COO⁻ (NH₄)⁺ aqueous solution having a metal content of 5 ppm or 50 ppm, had the high surface tension reducing ability and was clear. Especially, each of the Example samples 4 to 18 having a pH in a range.of from 2 to 12, had desirable properties such that even when the solid concentration was 30.0 mass%, it remained as a liquid.

On the other hand, each of the Comparative Example samples 1 to 3 which used a CF₃CF₂O(CF₂)₂OCF₂COO⁻(NH₄⁺) aqueous solution having a metal content of 250 ppm, had a low surface tension reducing ability as compared with each of the Example samples 4 to 6 under the same conditions except for the metal content.

### (C-1) PRODUCTION TEST FOR PTFE

### EXAMPLE c1

Into a 100 L stainless steel autoclave equipped with a baffle plate and a stirrer, 233 g of a CF₃CF₂O(CF₂)₂OCF₂COO⁻(NH₄)⁺ aqueous solution (solid concentration: 30.0% and pH: 5.7) obtained in Example 1, 872 g of paraffin wax (melting point: 52°C) and 59 L of deionized water were introduced. After the autoclave was flushed with nitrogen, the pressure was reduced, and the autoclave was pressurized with tetrafluoroethylene (TFE). While stirring it, the temperature was raised to 70°C. Then, the pressure was raised with TFE to 1.765 MPa, and 5.0 g of disuccinic acid peroxide (concentration: 80 mass% and the rest is water) was dissolved in 1 L warm water at 70°C and injected into the autoclave. In about 3 minutes, the inner pressure decreased to 1.746 MPa.

Polymerization was conducted with addition of TFE so as to keep the autoclave inner pressure at 1.765 MPa. During the polymerization, the CF₃CF₂O(CF₂)₂OCF₂COO⁻(NH₄)⁺ aqueous solution was added in a total of 417 g. Further, ammonium sulfite was dissolved in water and added during the polymerization in a total of 4 g as ammonium sulfite. In a halfway, the temperature was lowered to 64°C, and in the last half of the polymerization, it was raised to 80°C. When the amount of TFE added became 23 kg, the reaction was terminated, and TFE in the autoclave was released to the atmosphere. The polymerization time was 115 minutes. The obtained aqueous emulsion of PTFE was cooled, and the supernatant paraffin wax was removed. The solid content concentration of the aqueous emulsion was about 26 mass%. Further, the average primary particle size of PTFE fine particles was 0.275 µm. Coagulation in the reactor was only at a level of a trace.

Such an aqueous emulsion was diluted with pure water to a concentration of 10 mass% and was adjusted to a temperature of 20°C, followed by stirring to agglomerate PTFE fine particles. The obtained PTFE powder was dried in an oven at 180°C for 6 hours. The obtained PTFE powder was colorless white and had a standard specific gravity (SSG) of 2.151 and an average particle size of 550 µm.

Then, 50 g of the PTFE powder was added to 40 mL of a 13.6% nitric acid solution, followed by heating at 80°C for 2 hours. Further, 30 mL of an eluent was evaporated to dryness, and the residue was dissolved in the nitric acid solution whereupon the metal concentration in the solution was quantified by an ICP-MS method. The metal concentration was 15 ng/g.

### COMPARATIVE EXAMPLE c1

A PTFE powder was obtained by carrying out the same reaction as in Example c1 except that the CF₃CF₂O(CF₂)₂OCF₂COO⁻(NH₄)⁺ aqueous solution (solid concentration: 30.0% and pH: 5.7) in Example c1 was changed to the CF₃CF₂O(CF₂)₂OCF₂COO⁻(NH₄)⁺ aqueous solution (solid concentration: 30.0% and pH: 5.7) obtained in Comparative Example 1. The obtained PTFE powder was slightly colored. 50 g of the obtained PTFE powder was added to 40 mL of a 13.6% nitric acid solution, followed by heating at 80°C for 2 hours. Further, 30 mL of an eluent was evaporated to dryness, and the residue was dissolved in the nitric acid solution, whereupon the metal concentration in the solution was quantified by an ICP-MS method. The metal concentration was 80 ng/g.

### (C-2) TEST FOR PRODUCING TFE/PPVE COPOLYMER

### EXAMPLE c2

A 1.3 L polymerization tank equipped with a stirrer
was degassed, and into the tank, 600 g of deionized water having 3 g of the CF₃CF₂O(CF₂)₂OCF₂COO⁻(NH₄)⁺ aqueous solution (solid concentration: 30.0% and pH: 5.7) obtained in Example 1 dissolved therein, 1.0 g of methanol, 35 g of CF₂CFOCF₂CF₂CF₃ (perfluoro(propyl vinyl ether), hereinafter referred to as PPVE) an 0.1 g of ammonium persulfate (hereinafter referred to as APS) were introduced, followed by stirring with a stirring rotational speed of 300 rpm. Inside of the polymerization tank was raised to a temperature of 65°C, and tetrafluoroethylene (hereinafter referred to as TFE) was introduced therein. The pressure inside of the polymerization tank was adjusted to 1.0 MPa to start polymerization. In order to have a constant pressure during the polymerization, TFE was continuously introduced in the tank, and when the amount of TFE continuously introduced reached 200 g, inside of the polymerization tank was cooled to room temperature, and unreacted TFE was purged. The polymerization tank was opened, and the obtained aqueous emulsion was frozen and thawed to separate out a copolymer, followed by washing three times with 1,000 mL of deionized water (25°C). Then, the resultant was dried at 150°C for 12 hours to obtain 205 g of a TFE/PPVE copolymer. The obtained copolymer had a melting point of 305°C, a MFR (Melt Flow Rate) (372°C and a load of 5 kg) of 15 g/min and a content of a polymerized unit derived from PPVE in the copolymer of 3.9 mass% (1.47 mol%), and it was a melt-moldable fluororesin. Such a resin was hot-pressed at 340°C, and the Yellow Index of the obtained sheet (thickness: 1.5 mm) was measured by using a color-difference meter. The YI value=-6, whereby the sheet was confirmed to be colorless white.

### COMPARATIVE EXAMPLE c2

The same reaction as in Example c2 was carried out except that the CF₃CF₂O(CF₂)₂OCF₂COO⁻(NH₄)⁺ aqueous solution (solid concentration: 30.0% and pH: 5.7) obtained in Example c2 was changed to the CF₃CF₂O(CF₂)₂OCF₂COO⁻(NH₄)⁺ aqueous solution (solid concentration: 30.0% and pH: 5.7) obtained in Comparative Example 1, and the obtained fluororesin was hot-pressed at 340°C. The Yellow Index of the obtained sheet (thickness: 1.5 mm) was measured by using a color-difference meter, and the YI value=+3, whereby brown coloration was observed.

### (C-3) TEST FOR PRODUCING TFE/HFP COPOLYMER

### EXAMPLE c3

The same polymerization tank as in Example c2 was degassed, and into the tank, 600 g of deionized water having 3 g of the CF₃CF₂O(CF₂)₂OCF₂COO⁻(NH₄)⁺ aqueous solution (solid concentration: 30.0% and pH: 5.7) obtained in Example 1 dissolved therein, 200 g of hexafluoropropylene (hereinafter referred to as HFP) and 0.3 g of APS, were introduced, followed by stirring with a stirring rotational speed of 300 rpm. Inside of the polymerization tank was raised to a temperature of 65°C, and TFE was introduced therein. The pressure inside of the polymerization tank was adjusted to 1.5 MPa to start polymerization. In order to have a constant pressure during the polymerization, TFE was continuously introduced in the tank, and when the amount of TFE continuously introduced reached 150 g, inside of the polymerization tank was cooled to room temperature the unreacted monomer was purged. The polymerization tank was opened, and the obtained aqueous emulsion was frozen and thawed to separate out a copolymer, followed by washing three times with 1,000 mL of deionized water (25°C). Then, the resultant was dried at 150°C for 12 hours to obtain 160 g of a TFE/HFP copolymer. The obtained copolymer had a melting point of 261°C, a MFR (372°C and a load of 5 kg) of 17 g/min and a content of a polymerized unit derived from HFP in the copolymer of 11.8 mass% (7.9 mol%), and it was a melt-moldable fluororesin. Such a resin was hot-pressed at 340°C, and the Yellow Index of the obtained sheet (thickness: 1.5 mm) was measured by using a color-difference meter. The YI value=-6, whereby the sheet was confirmed to be colorless white.

### COMPARATIVE EXAMPLE c3

The same reaction as in Example c3 was carried out except that the CF₃CF₂O(CF₂)₂OCF₂COO⁻(NH₄)⁺ aqueous solution (solid concentration: 30.0% and pH: 5.7) obtained in Example c3 was changed to the CF₃CF₂O(CF₂)₂OCF₂COO⁻(NH₄)⁺ aqueous solution (solid concentration: 30.0% and pH: 5.7) obtained in Comparative Example 1, and the obtained fluororesin was hot-pressed at 340°C. The Yellow Index of the obtained sheet (thickness: 1.5 mm) was measured by using a color-difference meter, and the YI value=+3, whereby brown coloration was observed.

### (C-4) TEST FOR PRODUCING TFE/ETHYLENE/PFBE COPOLYMER

### EXAMPLE c4

The same polymerization tank as in Example c2 was degassed, and into the tank, 600 g of deionized water having the CF₃CF₂O(CF₂)₂OCF₂COO⁻(NH₄)⁺ aqueous solution (solid concentration: 30.0% and pH: 5.7) obtained in Example 1 dissolved therein, 60 g of tertiary butanol, 2.4 g of (perfluorobutyl)ethylene (hereinafter referred to as PFBE) and 0.15 g of APS, were introduced, followed by stirring with a stirring rotational speed of 300 rpm. 111 g of TFE and 8 g of ethylene (hereinafter referred to as E) were introduced into the tank, and inside of the polymerization tank was raised to a temperature of 65°C to start polymerization. The polymerization pressure was 2.9 MPa. In order to have a constant pressure during the polymerization, a monomer mixture having a molar ratio of TFE/E=53/47 was continuously introduced into the tank, and 0.3 g of PFBE was introduced every time when 10 g of the monomer mixture was introduced. When the amount of the monomer mixture continuously introduced therein reached 270 g, inside of the polymerization tank was cooled to room temperature, and unreacted monomer was purged. The polymerization tank was opened, and the obtained aqueous emulsion was frozen and thawed to separate out a copolymer, followed by washing three times with 1,000 mL of deionized water (25°C). Then, the resultant was dried at 150°C for 12 hours to obtain 285 g of a TFE/E copolymer. The obtained copolymer had a melting point of 262°C, a MFR (297°C and a load of 5 kg) of 8 g/min and a molar ratio of a polymerized unit derived from TFE in the copolymer/a polymerized unit derived from E/a polymerized unit derived from PFBE of 52.5/46.7/0.8, and it was a melt-moldable fluororesin. Such a resin was hot-pressed at 300°C, and the yellow index of the obtained sheet (thickness: 1.5 mm) was measured by using a color-difference meter. The YI value=-6, whereby the sheet was confirmed to be colorless white.

### COMPARATIVE EXAMPLE c4

The same reaction as in Example c4 was carried out except that the CF₃CF₂O(CF₂)₂OCF₂COO⁻(NH₄)⁺ aqueous solution (solid concentration: 30.0% and pH: 5.7) obtained in Example c4 was changed to the CF₃CF₂O(CF₂)₂OCF₂COO⁻(NH₄)⁺ aqueous solution (solid concentration: 30.0% and pH: 5.7) obtained in Comparative Example 1, and the obtained fluororesin was hot-pressed at 300°C. The yellow index of the obtained sheet (thickness: 1.5 mm) was measured by using a color-difference meter, and the YI value=+3, whereby brown coloration was observed.

### INDUSTRIAL APPLICABILITY

The perfluorocarboxylic acid salt of the present invention has a high surface tension reducing ability and is excellent in its stability, whereby it is excellent in functioning as a surfactant. Further, it has a low content of metal such as iron. Therefore, the perfluorocarboxylic acid salt composition of the present invention is useful for various applications as a polymerization emulsifier, a leveling agent such as wax, a blowing aid, an additive for forming stable foams and improving fire-extinguishing performance for foam fire extinguishing, a cleaner, a mold releasing agent, an anticorrosive agent, a latex stabilizer, an anti-fogging agent for a film for agriculture, a pigment dispersant, to improve wettability or permeability of e.g. ink, coating or resist, to impart a curable resin water resistance and oil resistance, and an antifouling agent.

The entire disclosure of JP 20060236515 filed on August 31, 2006 including specification, claims and summary is enclosed.

## Claims

1. A process for producing a perfluorocarboxylic acid salt having an iron content of at most 10 ppm by mass measured by ICP and represented by the following formula (1):
R^{F}(OCF(X¹)CF₂)ₖ₋₁OCF(X²)COO⁻M⁺ (1)
wherein R^{F} is a C₁₋₁₀ monovalent perfluorinated organic group, each of X¹ and X² is independently a fluorine atom or a trifluoromethyl group, k is an integer of at least 1, and M⁺ is an ammonium ion or an alkyl-substituted ammonium ion, which comprises a hydrolysis step of hydrolyzing a compound (2) represented by the following formula (2) with water by using a reaction apparatus equipped with a reactor in which at least its inner surface is made of a fluororesin, wherein the amount of water to be used is from 0.9 to 1.2 times by mol based on the compound (2), wherein the hydrofluoric acid content in the reaction product is adjusted to be at most 0.1 mass% and then the obtained reaction product is subjected to distillation for purification, to obtain a compound (3) represented by the following formula (3), and a salt-forming step of the compound (3) to obtain the perfluorocarboxylic acid salt:
R^{F}(OCF(X¹)CF₂)ₖ₋₁OCF(X²)COF (2)
R^{F}(OCF(X¹)CF₂)ₖ₋₁OCF(X²)COOH (3)
wherein R^{F}, X¹, X² and k are respectively the same as R^{F}, X¹, X² and k in the formula (1).

2. The process for producing the perfluorocarboxylic acid salt according to Claim 1, wherein the total content of sodium and potassium is at most 50 ppm by mass measured by ICP.

3. The process for producing the perfluorocarboxylic acid salt according to Claim 1 or 2, wherein the total content of calcium and magnesium is at most 10 ppm by mass measured by ICP.

4. The process for producing the perfluorocarboxylic acid salt according to any one of Claims 1 to 3, wherein the total content of iron, sodium, potassium, calcium, magnesium, chromium and nickel is at most 70 ppm by mass measured by ICP.

5. The process for producing the perfluorocarboxylic acid salt according to any one of Claims 1 to 4, wherein the hydrolysis is carried out by reacting the compound (2) without a solvent.

6. The process for producing the perfluorocarboxylic acid salt according to any one of Claims 1 to 5, wherein the formed hydrofluoric acid is discharged out of the reaction system by bubbling nitrogen in a hydrolysis reaction crude liquid.

7. The process for producing the perfluorocarboxylic acid salt according to any one of Claims 1 to 6, wherein X¹ and X² in the formulae (1), (2) and (3) are both fluorine atoms.

## Patentansprüche

1. Verfahren zur Herstellung eines Perfluorcarbonsäuresalzes mit einem Eisengehalt von höchstens 10 ppm, bezogen auf die Masse, gemessen durch ICP, und dargestellt durch die folgende Formel (1):
R^{F}(OCF(X¹)CF₂)ₖ₋₁OCF(X²)COO⁻M⁺ (1)
worin R^{F} eine C₁₋₁₀ einwertige perfluorierte organische Gruppe ist, X¹ und X² jeweils unabhängig voneinander ein Fluoratom oder eine Trifluormethylgruppe sind, k eine ganze Zahl von mindestens 1 ist, und M⁺ ein Ammoniumion oder ein alkyl-substituiertes Ammoniumion ist, welches einen Hydrolyseschritt des Hydrolysierens einer Verbindung (2), dargestellt durch die folgende Formel (2), mit Wasser unter Verwendung einer Reaktionsvorrichtung, ausgestattet mit einem Reaktor, in welchem mindestens dessen innere Oberfläche aus einem Fluorharz ist, wobei die Menge an zu verwendendem Wasser von dem 0,9 bis 1,2-fachen, bezogen auf Mol, basierend auf der Verbindung (2), beträgt, wobei der Fluorwasserstoffgehalt in dem Reaktionsprodukt eingestellt wird, höchstens 0,1 Masse-% zu sein, und anschließend das erhaltene Reaktionsprodukt einer Destillation zur Reinigung unterworfen wird, um eine Verbindung (3) zu erhalten, dargestellt durch die folgende Formel (3), und einen Salzbildungsschritt der Verbindung (3), um das Perfluorcarbonsäuresalz zu erhalten, umfasst:
R^{F}(OCF(X¹)CF₂)ₖ₋₁OCF(X²)COF (2)
R^{F}(OCF(X¹)CF₂)ₖ₋₁OCF(X²)COOH (3)
worin R^{F}, X¹, X² bzw. k die gleichen wie R^{F}, X¹, X² und k in der Formel (1) sind.

2. Verfahren zur Herstellung des Perfluorcarbonsäuresalzes gemäß Anspruch 1, wobei der Gesamtgehalt an Natrium und Kalium höchstens 50 ppm, bezogen auf die Masse, gemessen durch ICP, beträgt.

3. Verfahren zur Herstellung des Perfluorcarbonsäuresalzes gemäß Anspruchs 1 oder 2, wobei der Gesamtgehalt an Calcium und Magnesium höchstens 10 ppm, bezogen auf die Masse, gemessen durch ICP, beträgt.

4. Verfahren zur Herstellung des Perfluorcarbonsäuresalzes gemäß einem der Ansprüche 1 bis 3, wobei der Gesamtgehalt an Eisen, Natrium, Kalium, Calcium, Magnesium, Chrom und Nickel höchstens 70 ppm, bezogen auf die Masse, gemessen durch ICP, beträgt.

5. Verfahren zur Herstellung des Perfluorcarbonsäuresalzes gemäß einem der Ansprüche 1 bis 4, wobei die Hydrolyse durch Umsetzung der Verbindung (2) ohne ein Lösungsmittel durchgeführt wird.

6. Verfahren zur Herstellung des Perfluorcarbonsäuresalzes gemäß einem der Ansprüche 1 bis 5, wobei der gebildete Fluorwasserstoff aus dem Reaktionssystem ausgetragen wird, indem Stickstoff in die Rohflüssigkeit der Hydrolysereaktion eingeblasen wird.

7. Verfahren zur Herstellung des Perfluorcarbonsäuresalzes gemäß einem der Ansprüche 1 bis 6, wobei X¹ und X² in den Formeln (1), (2) und (3) jeweils Fluoratome sind.

## Revendications

1. Procédé pour produire un sel d'acide perfluorocarboxylique ayant une teneur en fer d'au plus 10 ppm en masse, mesurée par ICP, et représenté par la formule (1) suivante :
R^{F}(OCF(X¹)CF₂)ₖ₋₁OCF(X²)COO⁻M⁺ (1)
dans laquelle R^{F} est un groupe organique perfluoré monovalent en C₁-C₁₀, chacun de X¹ et X² est indépendamment un atome de fluor ou un groupe trifluorométhyle, k est un entier valant au moins 1, et M⁺ est un ion ammonium ou un ion ammonium alkyl-substitué, qui comprend une étape d'hydrolyse consistant à hydrolyser un composé (2) représenté par la formule (2) suivante avec de l'eau par utilisation d'un dispositif réactionnel équipé d'un réacteur dont au moins la surface intérieure est faite en une résine fluorée, procédé dans lequel la quantité d'eau à utiliser est de 0,9 à 1,2 moles par mole du composé (2), la teneur en acide fluorhydrique dans le produit réactionnel étant ajustée de façon à être d'au plus 0,1 % en masse, après quoi le produit réactionnel obtenu est soumis à une distillation pour être purifié, afin que soit obtenu un composé (3) représenté par la formule (3) suivante, et une étape de formation de sel du composé (3) pour obtenir le sel d'acide perfluorocarboxylique :
R^{F}(OCF(X¹)CF₂)ₖ₋₁OCF(X²)COF (2)
R^{F}(OCF(X¹)CF₂)ₖ₋₁OCF (X²)COOH (3)
où R^{F}, X¹, X² et k sont respectivement les mêmes que R^{F}, X¹, X² et k dans la formule (1).

2. Procédé pour produire un sel d'acide perfluorocarboxylique selon la revendication 1, dans lequel la teneur totale en sodium et potassium est d'au plus 50 ppm en masse, mesurée par ICP.

3. Procédé pour produire un sel d'acide perfluorocarboxylique selon la revendication 1 ou 2, dans lequel la teneur totale en calcium et magnésium est d'au plus 10 ppm en masse, mesurée par ICP.

4. Procédé pour produire un sel d'acide perfluorocarboxylique selon l'une quelconque des revendications 1 à 3, dans lequel la teneur totale en fer, sodium, potassium, calcium, magnésium, chrome et nickel est d'au plus 70 ppm en masse, mesurée par ICP.

5. Procédé pour produire un sel d'acide perfluorocarboxylique selon l'une quelconque des revendications 1 à 4, dans lequel l'hydrolyse est mise en oeuvre par réaction du composé (2) sans solvant.

6. Procédé pour produire un sel d'acide perfluorocarboxylique selon l'une quelconque des revendications 1 à 5, dans lequel l'acide fluorhydrique formé est évacué du système réactionnel par barbotage d'azote dans un liquide brut de réaction d'hydrolyse.

7. Procédé pour produire un sel d'acide perfluorocarboxylique selon l'une quelconque des revendications 1 à 6, dans lequel X¹ et X² dans les formules (1), (2) et (3) sont tous deux des atomes de fluor.
